# EUROPÄISCHE PATENTANMELDUNG

(11) **EP 0 674 872 A1**
(43) Veröffentlichungstag der Anmeldung: **04.10.1995**
(21) Anmeldenummer: 95250065.0
(22) Anmeldetag: 21.03.1995
(51) Int. Cl.: A61B 1/05, A61B 1/00

(54) **Vorrichtung zur Beobachtung einer Körperhöhle**

(30) Priorität: 24.03.1994 DE 4410821
(71) Anmelder: Ärztliche Mechanik Udo Lindeke & Sohn, D-10999 Berlin (DE)
(72) Erfinder: Lindeke, Carsten, D-12459 Berlin (DE)
(74) Vertreter: Porth, Hans-Joachim

(57) **Zusammenfassung**

Vorrichtung zur Beobachtung einer Körperhöhle in der Medizintechnik, die gute Qualität der Bilder, leichte Handhabbarkeit und hohe Lebensdauer ermöglicht. Als Lösung ist vorgesehen, daß wenigstens ein Kamerakopf (18) einer Chipkamera oder dgl. der Betrachtungs- und/oder Übertragungseinrichtung in einer zumindest einseitig hermetisch verschlossenen und in einem Trokar relativbeweglichen Schutzhülse (11) anordenbar ist.

## Beschreibung

Die Erfindung betrifft eine Vorrichtung nach dem Oberbegriff des Patentanspruch 1.

In der Medizintechnik werden Körperhöhlen in bekannter Weise mittels eines Endoskops beobachtet, wozu das Endoskop über einen Trokar der Körperhöhle zugeführt wird. Zur Übertragung eines Bildes auf einen Monitor wird das Endoskop okularseitig über einen Adapter mit einer Kamera, beispielsweise einer TV-Kamera, verbunden. Die Nachteile einer derartigen Anordnung sind insbesondere darin zu sehen, daß das mit der Körperhöhle in unmittelbaren Kontakt tretende Endoskop sterilisiert und vorzugsweise autoklaviert werden muß, was sehr häufig schon frühzeitig eine Beschädigung des optischen Systems des Endoskops zur Folge hat. Außerdem treten wegen des Adaptierens Lichtverluste auf, die die Qualität des Bildes mindern. Schließlich ist diese Anordnung kompliziert aufgebaut, kostenaufwendig und schlecht handhabbar.

Der Erfindung liegt die Aufgabe zugrunde, eine Vorrichtung der eingangs erwähnten Gattung zu schaffen, die hohe Bildqualität, leichte Handhabbarkeit und mit geringem Aufwand eine hohe Lebensdauer ermöglicht.

Erfindungsgemäß wird die Aufgabe dadurch gelöst, daß wenigstens ein Kamerakopf oder eine Chipkamera oder aber auch die Optik eines sonstigen Betrachtungselements in einer wenigstens einseitig hermetisch verschlossenen, im wesentlichen formstabilen Schutzhülse anordenbar sind und letztere in einem Trokar relativbeweglich ist. Der Kamerakopf, die Chipkamera oder die Optik kommen somit nicht in direkten Kontakt mit der Körperhöhle. Sie müssen demgemäß keiner die Elektronik oder Optik schädigende Sterilisation ausgesetzt werden. Die Schutzhülse dagegen kann nach dem Einsatz und nachdem der Kamerakopf oder dgl. aus der Schutzhülse entnommen wurde, problemlos autoklaviert werden.

In weiterer Ausgestaltung der Erfindung ist vorgesehen, daß die Schutzhülse als wenigstens partiell transparenter im wesentlichen starrer Hohlzylinder ausgebildet ist, dessen innerhalb des Trokars zur Körperhöhle weisendes freies Ende verschlossen ist. Wegen der Transparenz der Schutzhülse ist eine ungehinderte Betrachtung der Körperhöhle stets gegeben, ohne daß der Kamerakopf oder die Chipkamera in Kontakt mit der Körperhöhle treten muß.

Vorzugsweise ist die Transparenz durch ein Fenster im freien Ende gegeben, das derart angeordnet ist, daß das Licht von der Körperhöhle ungehindert zum Kamerakopf oder zur Chipkamera gelangen kann.

In besonderer Ausgestaltung ist das Fenster der Stirnfläche des freien Endes der Schutzhülse zugeordnet bzw. durch diese gebildet. Hierdurch ist ein einfacher Aufbau der Schutzhülse möglich und wird die sichere Positionierung des Kamerakopfs bzw. der Chipkamera zum Fenster erleichtert.

Das Fenster ist in einfachster Weise mittels einer planparallelen Platte aus transparentem Werkstoff verschlossen. Es kann aber auch mit einer Linse verschlossen sein, die als Vorsatzlinse zum Kamerakopf oder zur Chipkamera zusätzliche optische Effekte ermöglicht.

Die Platte oder Linse besteht vorzugsweise aus Glas und ist durch Sintern mit einem metallenen Mantel der Schutzhülse verbunden. Der hermetische Verschluß ist somit auch nach häufigem Autoklavieren stets gewährleistet. Zusätzliche Dichtungsmittel, die durch das Autoklavieren in Mitleidenschaft gezogen werden könnten, sind nicht erforderlich.

Die Platte oder Linse kann aber auch mittels druck- und wärmebeständiger Dichtungsmittel in das Fenster eingesetzt sein, wenn dies insbesondere wegen des Materials, aus dem die Platte oder Linse besteht, zweckmäßig ist.

Die sichere Fixierung der Platte oder Linse innerhalb des Mantels der Schutzhülse und die hermetische Abdichtung sind dadurch begünstigt, daß sie in einer Ringnut des Mantels angeordnet ist.

Der Kamerakopf oder die Chipkamera ist im Bereich des freien Endes der Schutzhülse und innerhalb derselben mittels einer Halte- und Verstelleinrichtung relativbeweglich und insbesondere axialverschieblich sowie fixierbar. Hierdurch kann zum einen der Kamerakopf oder die Chipkamera stets in einer für die Betrachtung optimale Position zum Fenster der Schutzhülse angeordnet werden. Zum anderen ist insbesondere dann, wenn das Fenster mittels einer Linse verschlossen ist, durch die Axialverschieblichkeit eine ggf. zusätzliche Fokusierung möglich.

Weitere Merkmale der Erfindung und deren Vorteile ergeben sich aus den übrigen Ansprüchen und der Beschreibung.

Die Erfindung soll nachfolgend an einem Ausführungsbeispiel näher erläutert werden. In der zugehörigen Zeichnung zeigen:
- Fig. 1: eine Seitenansicht der Vorrichtung schematisch, teilweise sowie teilweise im Schnitt und
- Fig. 2: eine Seitenansicht einer Aufnahme schematisch, teilweise im Schnitt dargestellt.

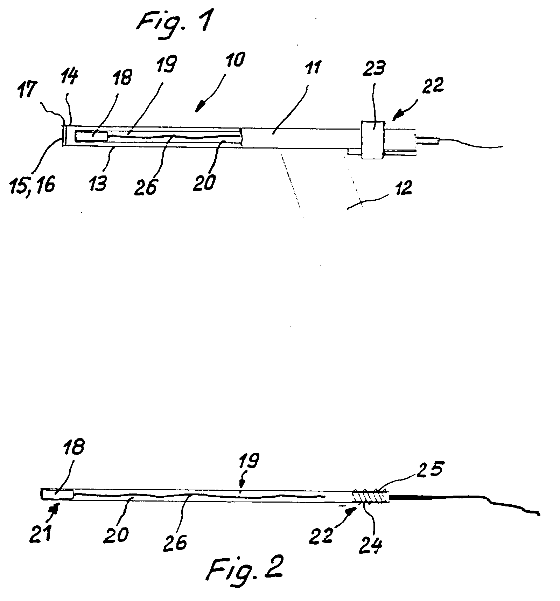

Eine Vorrichtung 10 zur Beobachtung von Körperhöhlen in der Medizientechnik, die über einen nicht dargestellten Trokar der zu beobachtenden Körperhöhle zugeführt wird, weist eine Schutzhülse 11 auf, der auf einer Seite ein Griff 12 zur Handhabung der Vorrichtung 10 zugeordnet ist.

Die Schutzhülse 11 hat einen dünnwandigen metallenen Mantel 13 und ist an einem freien, innerhalb des Trokars zur Körperhöhle weisenden Ende 14 mit einem Fenster 15 versehen und somit teilweise transparent. Letzteres ist der Stirnfläche des Endes 14 der Schutzhülse 11 zugeordnet bzw. durch die Stirnfläche selbst gebildet.

Das Fenster ist mittels einer Platte 16 aus Glas hermetisch verschlossen, wozu die Platte 16 in einer inneren Ringnut 17 des Mantels 13 gehalten ist. Die dichtmittelfreie Anordnung der Platte 16 in der Ringnut 13 erfolgt durch Sintern, so daß der hermetisch dichte Verschluß der Schutzhülse 11 auf der Seite des freien Endes 14 auch nach oftmaligem Sterilisieren der Schutzhülse 11 durch Autoklavieren sichergestellt ist.

Innerhalb der Schutzhülse 11 ist im vorliegenden Ausführungsbeispiel ein Kamerakopf 18 angeordnet, mittels dem die Beobachtung der Körperhöhle durch das Fenster 14 der Schutzhülse 11 bzw. die Übertragung der Bilder erfolgt. Als Kamerakopf 18 ist ein als sogenannte Gummilinse ausgebildetes oder aber auch ein nicht fokusierbares Objektiv einer Kamera und ggf. ein zugehöriger Kamerachip, folglich ein Teil einer Betrachtungs- und/oder Übertragungseinrichtung, bezeichnet. Es können in der Schutzhülse 11 aber auch koplette Kameras oder sonstige Betrachtungs- und/oder Übertragungseinrichtungen angeordnet sein, sofern sie eine entsprechende Dimensionierung aufweisen.

Der Kamerakopf 18 wird von einer Aufnahme 19 gehalten und ist mittels derselben in die Schutzhülse 11 einführbar sowie in dieser fixierbar. Die Aufnahme 19 weist eine Hülse 20 auf, deren freiem Ende 21 der Kamerakopf 18 zugeordnet ist. Von dem Kamerakopf 18 erstreckt sich innerhalb der Hülse 20 ein Übertragungskabel 26, das auf der dem Ende 21 gegenüberliegende Seite aus der Hülse 20 und der Schutzhülse 11 heraustritt und den Kamerakopf 18 mit der übrigen, nicht dargestellten, Betrachtungs- und/oder Übertragungseinrichtung verbindet. Die Hülse 20 wird in der Schutzhülse 11 so angeordnet, daß die freien Enden 14; 21 der Schutzhülse 11 wie auch Hülse 20 und somit das Fenster 14 der Schutzhülse 11 und der Kamerakopf 18 mit einander korrespondieren können.

Die Positionierung des Kamerakopfs 18 zum Fenster 14 erfolgt mittels einer Halte- und Verstelleinrichtung 22, die der Schutzhülse 11 und Hülse 20 auf den den freien Enden 14; 21 gegenüberliegenden Seiten zugeordnet ist. Die Halte- und Verstelleinrichtung 22 ist einerseits durch einen Stellring 23 gebildet, der in Axialrichtung unverschieblich aber drehbeweglich von der Schutzhülse 11 gehalten ist, wobei der Stellring 23 mit einem Innengewinde versehen ist. Andererseits ist die Halte- und Verstelleinrichtung 22 durch ein Gewindestück 24 gebildet, das drehfest mit der Hülse 20 der Aufnahme 19 verbunden ist.

Die Anordnung der Aufnahme 19 mit dem Kamerakopf 18 in der Schutzhülse 11 und die Feinpositionierung des Kamerakopfes 18 zum Fenster 14 geschieht in folgender Weise.

Zuerst wird der Kamerakopf 18 und das zugehörige freie Ende 21 der Hülse 20 auf der dem freien Ende 14 gegenüberliegenden Seite in die Schutzhülse 11 eingeführt. Dabei sind das Innengewinde des Stellrings 23 bzw. der Außendurchmesser der Hülse 20 so dimensioniert, daß die Hülse 20 den Stellring 23 ungehindert passieren kann. Die Hülse 20 wird sodann in die Schutzhülse 21 eingeschoben, bis der Gewindeansatz eines Außengewindes 25 des Gewindestücks 24 zur Anlage an den Gewindeansatz des Innengewindes des Stellrings 23 gelangt, wobei die Hülse 20 in der Schutzhülse 21 drehfest angeordnet, folglich allein axialverschieblich ist. Nachfolgend wird der Stellring 23 gedreht, bis das Gewindestück 24 etwa symetrisch im Stellring 23 aufgenommen ist. Hierdurch sind der Stellring 23 und das Gewindestück 24 derart zueinander angeordnet, daß der Kamerakopf 18 zum Fenster 14 annähernd in Arbeitsposition angeordnet ist. Ein weiteres Drehen des Stellrings 23 in die eine oder andere Richtung bewirkt eine weitere oder rückläufige Axialverschiebung des Kamerakopfs 18 innerhalb der Schutzhülse 11 und somit eine Feineinstellung der Arbeits- bzw. Aufnahmeposition des Kamerakopfes 18. Das Gewinde ist als selbsthemmendes Bewegungsgewinde ausgebildet, wobei zur Sicherung der Arbeitsposition des Kamerakopfes 18 dem Stellring 23 ein Halteorgan zum fixieren des Stellrings 23 zugeordnet sein kann. In das Fenster 14 kann anstelle der Platte 16 oder zusätzlich zu dieser eine Linse oder ein Linsensystem eingesetzt sein, so daß durch die Feineinstellung des Kamerakopfs 18 eine ggf. zusätzliche Fokusierung möglich ist. Mitunter ist wegen dieser Möglichkeit die Verwendung einer Kamera, die über eine Gummilinse verfügt und demzufolge allein eine Fokusierung ermöglicht, nicht erforderlich, woraus eine Kostenersparnis für die Vorrichtung 10 insgesamt resultieren könnte.

Nach der Beobachtung werden die Vorrichtung 10 aus dem Trokar herausgezogen und anschließend die Aufnahme 19 mit dem Kamerakopf 18 aus der Schutzhülse 11 entnommen, was umgekehrt zu der vorstehend beschriebenen Weise leicht möglich ist. Die mit der Körperhöhle in unmittelbaren Kontakt getretene Schutzhülse 11 kann sodann separat autoklaviert und für die nächste Beobachtung vorbereitet werden. Die empfindlichen Betrachtungs- und/oder Übertragungseinrichtungen, die aus der Schutzhülse 11 entfernt wurden, müssen einer solchen Prozedur nicht unterzogen werden, da sie während der Beobachtung durch die Schutzhülse 11 gegen die Körperhöhle gewissermaßen isoliert bzw. abgeschirmt waren. Eine Verwendung von kostenaufwendigen autoklavierbaren Optiken, Kameraköpfen und dgl. ist somit nicht erforderlich. Die Schutzhülse 11 begünstigt dagegen wegen ihres einfachen Aufbaus und der quasi unbegrenzten Wiederverwendbarkeit die Kosten für die Vorrichtung 10.

### Bezugszeichenaufstellung

- 10: Vorrichtung
- 11: Schutzhülse
- 12: Griff
- 13: Mantel
- 14: Ende
- 15: Fenster
- 16: Platte
- 17: Ringnut
- 18: Kamerakopf
- 19: Aufnahme
- 20: Hülse
- 21: Ende
- 22: Halte- und Verstelleinrichtung
- 23: Stellring
- 24: Gewindestück
- 25: Außengewinde
- 26: Übertragungskabel

## Patentansprüche

1. Vorrichtung zur Beobachtung einer Körperhöhle, mit einer der Körperhöhle über einen Trokar zuführbaren Betrachtungs- und/oder Übertragungseinrichtung, dadurch gekennzeichnet, daß wenigstens ein Kamerakopf (18), eine Chipkamera oder dgl. der Betrachtungs- und/oder Übertragungseinrichtung in einer zumindest einseitig hermetisch verschlossenen und im Trokar relativbeweglichen Schutzhülse (11) anordenbar ist.

2. Vorrichtung nach Anspruch 1, dadurch gekennzeichnet, daß die Schutzhülse (11) als wenigstens partiell transparenter im wesentlichen starrer Hohlzylinder ausgebildet ist, dessen innerhalb des Trokars zur Körperhöhle weisendes freies Ende (14) verschlossen ist.

3. Vorrichtung nach Anspruch 1 und 2, dadurch gekennzeichnet, daß die Transparenz der Schutzhülse (11) durch mindestens ein Fenster (15) im Bereich des freien Endes (14) gegeben ist, durch welches Licht zwischen der Körperhöhle und den Kamerakopf (18) leitbar ist.

4. Vorrichtung nach einem oder mehreren der Ansprüche 1 bis 3, dadurch gekennzeichnet, daß das Fenster (15) der Stirnfläche des freien Endes (14) der Schutzhülse (11) zugeordnet ist.

5. Vorrichtung nach einem oder mehreren der Ansprüche 1 bis 4, dadurch gekennzeichnet, daß ein Sichtfenster einem Mantel der Schutzhülse (11) zugeordnet ist.

6. Vorrichtung nach einem oder mehreren der Ansprüche 1 bis 5, dadurch gekennzeichnet, daß das Fenster (15) der Stirnfläche (11) mittels mindestens einer planparallelen Platte (16) oder einer Linse aus transparentem Werkstoff verschlossen ist.

7. Vorrichtung nach einem oder mehreren der Ansprüche 1 bis 6, dadurch gekennzeichnet, daß die Platte (16) oder Linse aus Glas besteht und diese durch Sintern mit dem Mantel der Schutzhülse (11) dichtmittelfrei verbunden ist.

8. Vorrichtung nach einem oder mehreren der Ansprüche 1 bis 7, dadurch gekennzeichnet, daß der Kamerakopf (18) mittels einer Aufnahme (19) innerhalb der Schutzhülse (11) relativbeweglich und fixierbar anordenbar ist.

9. Vorrichtung nach einem oder mehreren der Ansprüche 1 bis 8, dadurch gekennzeichnet, daß die Aufnahme (19) eine Hülse (20) aufweist und einem freien Ende (21) derselben, das mit dem freien Ende (14) der Schutzhülse (11) korrespondierend anordenbar ist, der Kamerakopf (18) zuordenbar ist.

10. Vorrichtung nach einem oder mehreren der Ansprüche 1 bis 9, dadurch gekennzeichnet, daß der Schutzhülse (11) und Hülse (20) auf der den freien Enden (14; 21) gegenüberliegenden Seite einer Halte- und Verstelleinrichtung (22) zur Axialverschiebung und Fixierung der Hülse (20) innerhalb der Schutzhülse (11) zugeordnet ist.
